# EUROPEAN PATENT SPECIFICATION

(11) **EP 0 409 302 B1**
(45) Date of publication and mention of the grant of the patent: **20.04.1994**
(21) Application number: 90201717.7
(22) Date of filing: 28.06.1990
(51) Int. Cl.: A61K 7/00

(54) **Cosmetics**
Kosmetische Präparate
Produits cosmétiques

(30) Priority: 19.07.1989 GB 8916508
(43) Date of publication of application: 23.01.1991
(73) Proprietor: Blendax GmbH, D-55120 Mainz (DE)
(72) Inventor: Busch, Northild, D-6228 Eltville 5 (DE); Rau, Werner, D-6501 Mommenheim (DE)

(56) References cited:
- EP-A- 0 342 924
- US-A- 2 773 834
- US-A- 4 172 140

## Description

The present invention relates to water-based cosmetics, preferably those for topical application, which contain a new, effective and skin-compatible preservative system.

Such cosmetics, in particular skin cleansers and hair cleansers such as foam-bath and shower-bath compositions, shampoos and also skin creams require preservation in order to be protected against microbial decomposition and, consequently, against deterioration. In the course of recent decades, numerous preservatives have been developed which it has also been possible to use in some cases for preserving cosmetics. In addition to having an adequate effectiveness against gram-positive and gram-negative bacteria and also against fungi, these preservatives must also, of course, be toxicologically absolutely safe and skin-compatible. This requirement is satisfied only by a few preservatives; it happens repeatedly that skin allergies and skin sensitizations occur after applying cosmetics containing preservatives.

There therefore continues to be a need to provide preservatives which are effective but do not exhibit the side effects described.

The present invention now provides a preservative system which is effective both against gram-positive and also gram-negative bacteria and also against fungi but has an excellent skin-compatibility and does not cause any allergies. This preservative system is composed of a mixture of 1,3-bis(hydroxymethyl)-5,5-dimethylhydantoin, an organic complexing agent and a water-soluble salt of benzoic acid. The cosmetic according to the invention contains 0.1-0.5% by weight of 1,3-bis(hydroxymethyl)-5,5-dimethylhydantoin, 0.05-0.5% by weight of an organic complexing agent, and 0.1-0.5% by weight of a water-soluble salt of benzoic acid, based in each case on the total composition of the cosmetic. The preferred concentration of 1,3-bis(hydroxymethyl)-5,5-dimethylhydantoin in the cosmetic according to the invention is between 0.1 and 0.3% by weight, in particular 0.13-0.3, most preferably, about 0.2% by weight. The use of this substance, which is also marketed under the tradename of "Glydant", as a preservative is already known and is described in various publications, for example in the Journal of the Society of Cosmetic Chemists, Vol. 31/2 (1980), pages 85-96; Soap, Cosmetics, Chemical Specialties, Vol. 62/3 (1986), pages 28-29, 78; and also "Cosmetic and Drug Preservation", Cosmetic Science and Technology Series, Volume 1, pages 165-190, which contains numerous literature references.

The second constituent of the preservative mixture contained in the cosmetic according to the invention is an organic complexing agent.

Used as such are, in particular, ethylenediaminetetraacetic acid (EDTA) and its water-soluble salts, in particular the di-, tri- and tetrasodium salts. Their proportion is preferably between 0.1 and 0.3, in particular 0.1 and 0.2, and particularly preferably, about 0.15% by weight of the total composition, based on free acid. Instead or the EDTA or its alkali-metal salts which are preferably used, use may, of course, also be made of other complexing agents in corresponding amounts.

As such, mention may be made, in particular, of nitrilotriacetic acid (NTA), nitrilotriphosphonic acid (NTP), ethanehydroxydiphosphonic acid (EHDP) and the alkylenediaminotetra(methylenephosphonic acids), in particular ethylenediaminotetramethylenephosphonic acid (EDATMPA) and also their alkali-metal salts, in particular, the sodium salts. The proportion of these complexing agents is within the preferred ranges specified above for EDTA.

The fact that complexing agents are able to increase the antimicrobial effectiveness of antimicrobial agents, is known for many of these substances. US Patent Specification 4,172,140 has already disclosed preservative mixtures for technical purposes such as cutting oils, lubricants, liquids for metal machining, etc., which contain a condensation product of 5,5-dimethylhydantoin with formaldehyde in a ratio by weight of 1:1-2 and an alkali-metal salt of EDTA. These compositions are not, however, recommended for use in cosmetics and also do not contain the third constituent, which is essential according to the invention, a water-soluble salt of benzoic acid. The third constituent of the preservative mixture in the cosmetics according to the invention is a water-soluble benzoic acid salt. This is contained preferably in an amount of between 0.15 and 0.40% by weight, in particular of 0.20 to 0.30, and particularly preferably, about 0.25% by weight, based on the total composition of the cosmetic. The preferred benzoic acid salt is sodium benzoate, but for example, potassium benzoate, lithium benzoate, calcium benzoate, magnesium benzoate etc., are also suitable, the optimum concentrations suitable above for sodium benzoate having to be correspondingly varied upwards.

The cosmetics according to the invention are preferably skin cleansers and hair cleansers and also water-based skin creams.

Such products are, for example, hair shampoos, foam-bath and shower-bath compositions, moisture creams, sun-protection creams, hand creams etc., which may be packaged, if desired and necessary, also as aerosol foams using standard aerosol propellants such as dimethyl ether or low hydrocarbons such as propane, butane and isobutane.

The composition of such cosmetics is in principle known and is described in the standard cosmetic works, for example K. Schrader, Grundlagen und Rezepturen der Kosmetika [Principles and Formulations of Cosmetics] (1979, published by Dr. Alfred Hüthig Verlag, Heidelberg), W. Umbach, Kosmetik - Entwicklung, Herstellung und Anwendung kosmetischer Mittel [Cosmetics - Development, Production and Application of Cosmetics] (1988, published by Georg Thieme Verlag, Stuttgart), and G.A. Nowak, Die kosmetischen Präparate [Cosmetic Preparations], 3rd edition, in particular volume 2 (1984, published by Verlag für Chemische Industrie Ziolkowsky, Augsburg).

A few examples of cosmetics according to the invention which contain the preservative mixture described are given below.

| 1. Egg shampoo | |
|---|---|
| C₁₂-C₁₄-Alkyl ether sulphate, sodium salt | 12.00 (% by weight) |
| Coconut fatty acid diethanolamide | 1.50 |
| Fatty alcohol polyglycol ether | 1.00 |
| Protein hydrolysate | 0.80 |
| Egg yolk | 0.75 |
| Perfume | 0.30 |
| Sodium chloride | 2.30 |
| 1,3-Bis(hydroxymethyl)-5,5-dimethylhydantoin (Glydant^{·}) | 0.37 |
| EDTA, tetrasodium salt | 0.15 |
| Sodium benzoate | 0.25 |
| Yellow dyestuff | q.s |
| Water | to 100.00 |

| 2. Conditioning shampoo | |
|---|---|
| C₁₂-C₁₄-Alkyl ether sulphate, sodium salt | 10.00 (% by weight) |
| Quaternary cellulose derivative (polymer JR 125) | 0.50 |
| EDTA, tetrasodium salt | 0.20 |
| 1,3-Bis(hydroxymethyl)-5,5-dimethylhydantoin | 0.25 |
| Sodium benzoate | 0.30 |
| C₁₂-C₁₄-Fatty acid amidopropylbetain | 2.50 |
| Perfume | 0.30 |
| Dyestuff | q.s. |
| Water | to 100.00 |

| 3. Children's foam bath | |
|---|---|
| C₁₂-C₁₄-Alkyl ether sulphate, sodium salt | 12.50 (% by weight) |
| C₁₂-C₁₄-Alkyl ether sulphate, triethanolamine salt | 7.50 |
| Triethanolamine lauryl sulphate | 12.50 |
| Fatty acid-protein condensate | 2.50 |
| Perfume | 2.00 |
| Fatty acid monoglyceride | 1.50 |
| Glydant^{·} | 0.20 |
| EDTA, disodium salt | 0.20 |
| Sodium benzoate | 0.25 |
| Dyestuff | q.s. |
| Water | to 100.00 |

| 4. Camomile shampoo | |
|---|---|
| C₁₂-C₁₄-Alkyl ether sulphate, sodium salt | 15.00 (% by weight) |
| Coconut acid monoethanolamide | 2.00 |
| Sodium chloride | 2.00 |
| Camomile extract | 1.10 |
| Perfume | 0.50 |
| 1,3-Bis(hydroxymethyl)-5,5-dimethylhydantoin | 0.30 |
| EDATMPA, trisodium salt | 0.30 |
| Sodium benzoate | 0.20 |
| Green dyestuff | q.s. |
| Water | to 100.00 |

| 5. Herbal foam bath | |
|---|---|
| Sodium lauryl ether sulphate | 9.50 (% by weight) |
| Coconut acid diethanolamide | 0.50 |
| Sodium chloride | 3.00 |
| Plant extract | 1.00 |
| Glydant^{·} | 0.25 |
| EDTA, tetrasodium salt | 0.15 |
| Sodium benzoate | 0.25 |
| Perfume | 1.00 |
| Green dyestuff | q.s. |
| Water | to 100.00 |

| 6. Shower bath | |
|---|---|
| Sodium lauryl ether sulphate | 12.00 (% by weight) |
| Potassium salt of a fatty acid/tetrapeptide condensate | 4.50 |
| Ethoxylated castor oil | 0.50 |
| Quaternized cellulose ether (according to US Patent Specification 3,816,616) | 1.00 |
| Sodium chloride | 0.20 |
| Perfume | 1.50 |
| 1,3-Bis(hydroxymethyl)-5,5-dimethylhydantoin | 0.30 |
| EHDP, disodium salt | 0.13 |
| Sodium benzoate | 0.30 |
| Plant extract | 0.80 |
| Dyestuff | q.s. |
| Water | to 100.00 |

| 7. Sun-protection cream | |
|---|---|
| Polyethylene oxide fatty acid ester | 6.00 (% by weight) |
| Sorbitan fatty acid ester | 1.50 |
| Paraffin oil | 14.00 |
| Isopropyl isostearate | 6.00 |
| Ethylhexyl p-methoxycinnamate | 2.50 |
| Glycerol | 2.00 |
| 1,2-Propylene glycol | 1.80 |
| Magnesium sulphate heptahydrate | 0.70 |
| Perfume | 0.30 |
| 1,3-Bis(hydroxymethyl)-5,5-dimethylhydantoin | 0.13 |
| EDTA, tetrasodium salt | 0.13 |
| Sodium benzoate | 0.25 |
| Water | to 100.00 |

| 8. Face milk (water-in-oil emulsion) | |
|---|---|
| Polyethylene oxide fatty acid ester | 5.80 (% by weight) |
| Glycerol sorbitan fatty acid ester | 2.20 |
| C₈-C₁₂-Fatty acid triglyceride | 8.00 |
| Decyl oleate | 5.00 |
| 2-Octyldodecanol | 3.00 |
| Squalane | 6.00 |
| Glycerol | 2.00 |
| 1,2-Propylene glycol | 1.80 |
| Magnesium sulphate heptahydrate | 0.70 |
| Perfume | 0.30 |
| Glydant^{·} | 0.15 |
| EDTA (Trilon^{·}B) | 0.10 |
| Sodium benzoate | 0.30 |
| Water | to 100.00 |

The microbiological investigations described below show the effectiveness of the preservative system used according to the invention. In these investigations, two products with identical compositions in each case were preserved on the one hand, with the preservative system according to the invention and on the other, with a preservative hitherto normally used for such products, a mixture of 0.19% of 2-methyl-3-(2H)isothiazolone and 0.51% of 5-chloro-2-methyl-3-(2H)isothiazolone in benzyl alcohol (hereinafter termed "preservative A") and subjected to a standard microbial exposure test. The following results were obtained:

| 1. Product: camomile shampoo | | |
|---|---|---|
| Preservative | | Result |
| Glydant^{·} | 0.1% | Passed; suitable for production |
| Trilon^{·}B | 0.1% | |
| Sodium benzoate | 0.2% | |
| Glydant^{·} | 0.2% | Passed; suitable for production |
| Trilon^{·}B | 0.13% | |
| Sodium benzoate | 0.25% | |
| Glydant^{·} | 0.3% | Passed; suitable for production |
| Trilon^{·}B | 0.13% | |
| Sodium benzoate | 0.25% | |
| Preservative A | | Failed; not suitable for production |

| 2. Product: 25% solution of sodium C₁₂-C₁₄-alkyl ether sulphate | | |
|---|---|---|
| Preservative | | Result |
| Glydant^{·} | 0.13% | Passed; suitable for production |
| Trilon^{·}B | 0.13% | |
| Sodium benzoate | 0.25% | |
| Glydant^{·} | 0.2% | Passed; suitable for production |
| Trilon^{·}B | 0.13% | |
| Sodium benzoate | 0.25% | |
| Glydant^{·} | 0.3% | Passed; suitable for production |
| Trilon^{·}B | 0.13% | |
| Sodium benzoate | 0.25% | |
| Glydant^{·} | 0.37% | Passed; suitable for production |
| Trilon^{·}B | 0.13% | |
| Sodium benzoate | 0.25% | |
| Preservative A | | Failed; not suitable for production |

The results of these investigations show that the preservative system of the composition according to the invention is superior in its antimicrobial effectiveness to that of a commercially standard preservative for cosmetics without causing allergies. If, however, to increase the effectiveness, the concentration of the commercially standard "preservative A" were increased, the occurrence of such allergies on a considerable scale would be to be expected.

### Performance of the microbial exposure test:

Microbe-free products are artificially contaminated with microorganisms and examined after being stored for two and four weeks. The test is considered to have been passed and the product under test is considered "suitable for production" if, in the case of the two-week test, the product contains less than 1,000 CFU/g or ml and the bacterial count exhibits at least a constant or a falling trend up to the termination of the investigations after a total of 28 days. Products which contain more than 1,000 microbes per g or ml during the test period at the fourteen-day assessment or whose bacterial count exhibits a rising trend after termination of the test, i.e. after a total of four weeks, are always classified as "not suitable for production" and have therefore failed the test. In the tests carried out a mixture of 14 different microorganisms from the DSM (German Collection of Microorganisms) were used.

## Claims

1. Water-based cosmetic, characterized in that it contains 0.1-0.5% by weight of 1,3-bis(hydroxymethyl)-5,5-dimethylhydantoin, 0.05-0.5% by weight of an organic complexing agent, and 0.1-0.5% by weight of a water-soluble salt of benzoic acid, based in each case on the total composition of the cosmetic.

2. Cosmetic according to Claim 1, characterized in that it contains 0.1-0.3% by weight, based on the total composition of the cosmetic, of 1,3-bis(hydroxymethyl)-5,5-dimethylhydantoin.

3. Cosmetic according to Claim 1 or 2, characterized in that it contains 0.1-0.3% by weight of an organic complexing agent, based on the total composition of the cosmetic.

4. Cosmetic according to Claim 3, characterized in that it contains ethylenediaminetetraacetic acid or its alkali-metal salts as complexing agents.

5. Cosmetic according to one or more of the preceding claims, characterized in that it contains 0.15-0.40% by weight of a water-soluble benzoic acid salt, based on the total composition of the cosmetic.

6. Cosmetic according to one or more of the preceding claims, characterized in that it contains sodium benzoate as water-soluble benzoic acid salt.

## Patentansprüche

1. Kosmetische Präparate auf wäßriger Basis, dadurch gekennzeichnet, daß sie 0,1 bis 0,5 Gew.-% 1,3-Bis(hydroxymethyl)-5,5-dimethylhydantoin, 0,05 bis 0,5 Gew.-% eines organischen Komplexierungsmittels und 0,1 bis 0,5 Gew.-% eines wasserlöslichen Salzes der Benzoesäure, jeweils bezogen auf die Gesamtzusammensetzung der kosmetischen Präparate, enthalten.

2. Kosmetische Präparate nach Anspruch 1, dadurch gekennzeichnet, daß sie 0,1 bis 0,3 Gew.-%, bezogen auf die Gesamtzusammensetzung der kosmetischen Präparate, an 1,3-Bis(hydroxymethyl)-5,5-dimethylhydantoin enthalten.

3. Kosmetische Präparate nach Anspruch 1 oder 2, dadurch gekennzeichnet, daß sie 0,1 bis 0,3 Gew.-%, bezogen auf die Gesamtzusammensetzung der kosmetischen Präparate, an einem organischen Komplexierungsmittel enthalten.

4. Kosmetische Präparate nach Anspruch 3, dadurch gekennzeichnet, daß sie als Komplexierungsmittel Ethylendiamintetraessigsäure oder ihre Alkalimetallsalze enthalten.

5. Kosmetische Präparate nach einem oder mehreren der vorstehenden Ansprüche, dadurch gekennzeichnet, daß sie 0,15 bis 0,40 Gew.-%, bezogen auf die Gesamtzusammensetzung der kosmetischen Präparate, an einem wasserlöslichen Benzoesäuresalz enthalten.

6. Kosmetische Präparate nach einem oder mehreren der vorstehenden Ansprüche, dadurch gekennzeichnet, daß sie als wasserlösliches Benzoesäuresalz Natriumbenzoat enthalten.

## Revendications

1. Produit cosmétique à base aqueuse, caractérisé en ce qu'il contient 0,1 à 0,5% en poids de 1,3-bis(hydroxyméthyl)-5,5-diméthylhydantoïne, 0,05 à 0,5% en poids d'un agent complexant organique, et 0,1 à 0,5% en poids d'un sel soluble dans l'eau d'acide benzoïque, rapportés dans chaque cas à la composition totale du produit cosmétique.

2. Produit selon la revendication 1, caractérisé en ce qu'il contient 0,1 à 0,3% en poids, rapporté à la composition totale du produit cosmétique, de 1,3-bis(hydroxyméthyl)-5,5-diméthylhydantoïne.

3. Produit cosmétique selon la revendication 1 ou 2, caractérisé en ce qu'il contient 0,1 à 0,3% en poids d'un agent complexant organique, rapporté à la composition totale du produit cosmétique.

4. Produit cosmétique selon la revendication 3, caractérisé en ce qu'il contient de l'acide éthylènediaminetétraacétique ou ses sels de métaux alcalins en tant qu'agents complexants.

5. Produit cosmétique selon une ou plusieurs des précédentes revendications, caractérisé en ce qu'il contient 0,15 à 0,40% en poids d'un sel soluble dans l'eau de l'acide benzoïque, rapporté à la composition totale du produit cosmétique.

6. Produit cosmétique selon une ou plusieurs des précédentes revendications, caractérisé en ce qu'il contient du benzoate de sodium comme sel soluble dans l'eau de l'acide benzoïque.
